# EUROPEAN PATENT SPECIFICATION

(11) **EP 2 887 936 B1**
(45) Date of publication and mention of the grant of the patent: **23.03.2016**
(21) Application number: 13802433.6
(22) Date of filing: 24.09.2013
(51) Int. Cl.: A61K 31/28, C09C 1/36, A61P 31/00, A61K 45/06

(54) **ANTIMICROBIAL TITANIUM DIOXIDE NANOPARTICLES FUNCTIONALIZED WITH CATIONIC SILVER IONS**
MIT KATIONISCHEN SILBERIONEN FUNKTIONALISIERTE ANTIMIKROBIELLE TITANDIOXIDNANOPARTIKEL
NANOPARTICULES DE DIOXYDE DE TITANE ANTIMICROBIENNES FONCTIONNALISÉES AVEC DES IONS D'ARGENT CATIONIQUE

(43) Date of publication of application: 01.07.2015
(73) Proprietor: Pavia Farmaceutici S.r.l., 27010 Copiano (Pavia) (IT)
(72) Inventor: BIGNOZZI, Carlo Alberto, I-44121 Ferrara (IT); DISSETTE, Valeria, I-45030 Occhiobello (Rovigo) (IT); DI LALLO, Giuliana, I-55060 Massa Macinaia (Lucca) (IT); FERRARI, Massimo, I-27010 Copiano (Pavia) (IT)
(74) Representative: Biggi, Cristina
(86) International application number: PCT/IB2013/058805
(87) International publication number: WO 2015/044707

(56) References cited:
- WO-A1-2008/043396

## Description

The present invention relates in general term to Titanium dioxide derivatives, functionalized with silver cations by a bifunctional mercapto alkylsilane ligand. The present derivatives are particularly useful e.g. as antimicrobic, antiseptic and/or antiviral agent.

### Background of the art

The antibacterial activity of the silver Ag⁺ ions is well known in the art since a long time. To this extent, are also known pharmaceutical compositions or medicaments based on silver ions, e.g. silver sulfadiazine, used, for instance, to prevent infections in cases of severe burns, or, in general, as antibacterial agents. Prior studies (see, e.g., Carr, H. S., Wlodkowski, T.J., and Rosenkranz, H. S., 1973, "Antimicrobial agents and chemotherapy", vol. 4, p. 585) have demonstrated that the antibacterial activity of Ag⁺ ions is directly proportional to their concentration.

On the other hand, it is also known that elemental silver and silver salts usually demonstrate substantially less effectiveness against microbes. In the past, silver salt solutions, such as silver nitrate, were used to bathe the wound. This kind of administration, often requires large amounts of substance, in order to achieve a remarkable antibacterial effect. Ag⁺ based formulations are applied in pharmaceutical preparations, specially for topical use. However, most of the formulations based on silver salts, e.g. silver sulphadiazine or silver nitrate, suffer of photochemical instability.

Colloidal silver nanoparticles have also been a focus of increasing interest, and are being heralded as an excellent candidate for therapeutic purposes. Also such colloidal preparations are, however, photochemically unstable, to such an extent that even a brief exposure to visible light can cause the photoreduction of Ag⁺ to Ag° with a consequent breakdown of the antimicrobial action. Association of silver cations to titanium or silicon dioxide nanoparticles, has previously been described as reported for instance in WO2007/122651. In this publication there is described the stabilization of silver cations on a metal oxide surface, by the use of a bifunctional ligand. The described ligands provided indeed a binding to the metal oxide preventing at the same time the photoreduction of the silver cations to atomic silver, with a percentage of bound silver ions of about 0.2 % w/w. Such derivatives were prepared with synthetic routes that can take prolonged reaction times, and final products based on TiO₂ particles can be sometimes associated to a a peculiar smell.

It appears, therefore, that there is still the need to find antimicrobial TiO₂ derivatives, functionalized with a high content of silver ions and that can be rapidly and cheaply obtained, in a reliable and efficient way.
The Applicant has surprisingly found that when a specific mercapto alkyl silane ligand is used, it is possible to obtain a series of TiO₂ derivatives of formula (I), endowed with high antimicrobic, antiseptic and/or antiviral activity, thus overcoming the drawbacks of the state of art.

### Summary of the invention

In a first aspect, the invention relates to a TiO₂ functionalized derivative of formula (I):

**TiO₂-[L]-Ag⁺** **(I)**

wherein:
- -[L]-: is a bivalent mercapto alkyl silane ligand of general formula: -[Si-R-S]-, wherein R is a bivalent C₁-C₄ linear or branched alkyl group, optionally substituted;
- TiO₂: represents a titanium dioxide particle linked to the silica atom of the ligand -[L]-;
- Ag⁺: represents silver ions linked to the sulphur atom of the bivalent ligand -[L]-.

In a further aspect, the invention also refers to a **process** for the preparation of the above identified derivatives of formula (I), by reaction of titanium dioxide with a trialkyloxy mercapto alkyl silane derivative of formula (II), in the presence of a silver salt (III): 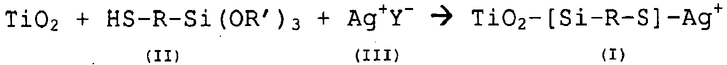 wherein:
- R: is as above defined;
- R': is independently selected from C₁-C₄ monovalent linear or branched alkyl group, optionally substituted;
- Y-: is an anionic counterion, preferably selected from: perchlorate (ClO₄⁻), acetate (CH₃COO⁻), and nitrate (NO₃⁻).

In another aspect, the invention refers to a derivative of general formula (I), for use as a **medicament,** in particular as antibacterial, antimycotic, antiviral and/or sporicidal agent.

In another aspect, the present invention also relates to a **pharmaceutical composition** comprising the above indicated derivatives of formula (I), in admixture with at least one physiologically acceptable excipient or carrier, and optionally, with another active ingredient. In particular, said composition can be in the form of a cream, a gel or a powder. This composition is suitable for the topical administration route, mainly intended for the use as antibacterial, antimycotic and antiviral agent.

Finally the description describes also a **method** for treatment of a mammal, including man, comprising the topical administration of an effective amount of a derivative of formula (I), or of a pharmaceutical composition thereof.

### Description of the drawings

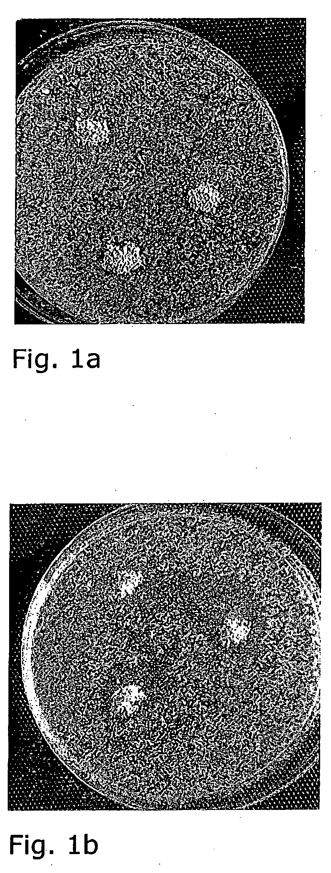

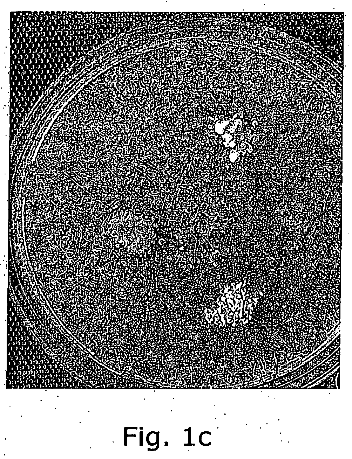

Figure 1 is a Petri capsule showing the inhibition of microbial colonies surrounding of the preferred derivative of the invention of formula TiO₂-MPS-Ag⁺, wherein:
A) TiO₂-MPS-Ag⁺ powder with a 0.58% Ag⁺ content.
B) TiO₂-MPS-Ag⁺ powder with 2.30% Ag⁺ content.
Figure 1C is a Petri capsule showing the control TiO₂ powder.

### Detailed description

The term "bivalent C₁-C₄ linear or branched alkyl group" means a linear or branched alkyl moiety having 1 to 4 carbon atoms, optionally substituted, which is linked to both the end sides to the rest of the molecule. Example of said bivalent group are: methylene (-CH₂-), ethylene (-CH₂CH₂-), propylene (-CH₂CH₂CH₂-).
Likewise, "monovalent C₁-C₄ linear or branched alkyl group", means a linear or branched alkyl moiety having 1 to 4 carbon atoms, optionally substituted, bonded to the rest of the molecule by one side, such as for examples: methyl (-CH₃), ethyl (-CH₂CH₃), propyl (-CH₂CH₂CH₃) and the like.

As above indicated, the invention deals with titanium dioxide particles, functionalized by silver ion, through a divalent mercapto alkyl silane ligand. The present derivatives are, in fact, characterized by having such a divalent ligand -[L]- of general formula: -[Si-R-S]-, which is able to connect the titanium dioxide particles (TiO₂) to silver ions (Ag⁺), thus forming the derivatives (I) of the invention. The chemical structure of the ligand, in fact, allows the binding to the titanium oxide on one side (via the Si atom), as well as the coordination with the silver ions on the other side, via the sulfur atom of the mercapto (-SH) group. In particular, the silica atom is linked to the titanium atom of the TiO₂ particle via bridging oxygen atoms; whereas the sulphur atom of the -SH moiety is connected to Ag⁺ ions, typically by means of coordination bonds.
By that, the TiO₂ particles can be functionalized with a high amount of silver ions, as below described in detail, in a stable and reliable way, thus forming the present derivatives of formula (I), endowed with the desired pharmaceutical action.

Preferably, in the derivatives (I), the R group is a methylene or, more preferably, a propylene residue, whereby the ligand -[L]- thus has the preferred formulae: -Si-CH₂-S- or -Si-CH₂CH₂CH₃-S-, being this latter even more preferred.
It follows that a preferred derivative of the invention is the compound (I) of formula: Ti-Si-CH₂CH₂CH₃-S-Ag⁺, herein also indicated as TiO₂-MPS-Ag⁺.
As far as the anionic titanium dioxide particles are concerned, it is to be noted that TiO₂ may exist in different allotropic forms: anastase, rutile and brookite, all of them commercially available, and suitable for the present invention. The oxide surface is nevertheless characterised by the presence of Ti-OH groups which may suitably react with the selected trialkyloxy mercapto alkyl silane derivative, thus linking the Si atom via bridging oxygen, and giving the derivatives of the invention.
In a preferred form, the derivatives (I) have a TiO₂ particle size comprised from 100nm to 600nm, more preferably comprised from 300 to 500 nm.

Advantageously, the derivatives of the invention are also characterized by having a high percentage of Ag⁺ ions, even up to 3% w/w, more preferably comprised from 0.5 to 3% w/w, wherein the % w/w represents the amount of Ag⁺ over the total weight. In fact, Applicant has found that by varying the amount of silver salt and mercapto trimethoxyalkilsilane derivatives of choice, it is possible to obtain the derivative (I) with the desired amount of silver ions. This means that the present derivatives can be readily used as antimicrobic, antiseptic and/or antiviral agent, even at a low dosage, nevertheless exhibiting a remarkable pharmaceutical action.

In fact, the derivatives (I) present a high surface coverage of the ligand -[L]- on the metal oxide surface, which allows to increase the amount of bounded silver ions, up to 1% w/w and even more. Even further, due to the fast binding reaction of the mercapto alkyl silane ligand -[L]- to the titanium oxide surface, it is possible to prepare the derivatives of the invention in low reaction times, such as in a 20-30 minutes range, as herein below described in detail. It follows that the present invention also addresses the problem of how to find an effective antibacterial agent having a high content of silver ions, which can be prepared in a reliable, cheap and quick way. In this direction, it is worth noting that the Applicant has conducted similar experiments also considering SiO₂ particles, in place of the TiO₂ of the invention. The results showed that, under the same experimental conditions, the use of the silica dioxide did not allow the preparation of the corresponding silver functionalized silica derivatives, of hypothetical formula SiO₂-[L]-Ag⁺. In fact, whilst the functionalized TiO₂ the derivatives (I) of the invention can be obtained in a very short frame of time, also endowed with a high antimicrobial activity (as shown in the experimental part), the use of SiO₂ in lieu of the TiO₂, on the contrary, did not result in any substantial formation of the corresponding silica derivatives. Even further, Applicant has found that the use of SiO₂ led to the formation of an undefined precipitate, only after more than 30 hours. Most importantly, said undefined precipitate did not show any remarkable antimicrobial activity.

Conveniently, the derivatives (I) can be prepared e.g. by purchasing the components (titanium dioxide, the selected ligand and the silver salt), and by following the synthetic process as below reported and described in detail.

In this sense, and according to a further aspect, the invention deals with a process for the preparation of the present derivatives of formula (I), by reaction of titanium dioxide with a trialkyloxy mercapto alkyl silane derivative of formula (II), in the presence of a silver salt (III), as generally illustrated in the following reaction scheme: 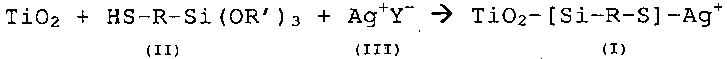 wherein:
- R: is as above defined;
- R': is independently selected from C₁-C₄ monovalent linear or branched alkyl group, optionally substituted;
- Y⁻: is an anionic counterion, preferably selected from: perchlorate (ClO₄⁻), Acetate (CH₃COO⁻), and nitrate (NO₃⁻).

The process can be expediently carried out at room temperature (e.g. comprised from about 15° C to about 40° C), preferably in a polar organic solvent, even more preferably, in acetone. The reaction can be monitored using technique and procedures known in synthetic chemistry such as atomic spectroscopy and the like.

The silver salt is generally selected among those salts that are soluble in polar organic solvents. According to an embodiment of the invention, the silver salt (III) is preferably selected from: silver perchloride (AgClO₄, CAS No. 7783-93-9) and silver nitrate (AgNO₃, CAS No. 7761-88-8).

As far as the trialkyloxy mercapto alkyl silane derivative of formula (II) is concerned, this is preferably a derivative of formula (II) wherein R is selected from: methylene (-CH₂-) and propylene (-CH₂CH₂CH₂-).
Especially preferred is the compound of formula (II) wherein R is a propylene (-CH₂CH₂CH₂-) residue and R' is methyl (-CH₃), i.e. the 3-mercaptopropyltrimethoxysilane compound of formula:

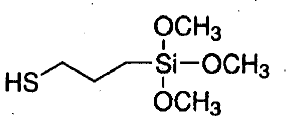

Equally preferred are compounds of formula (II) selected from: mercaptomethylmethyldietoxysilane, 3-mercaptopropylmethyldimethoxysilane and 3-mercaptopropyltriethoxysilane.

Practically, the present process for the preparation of the antimicrobial derivatives of formula (I), can be carried out by suspending the TiO₂ in the selected polar solvent, preferably acetone, and then adding thereto the selected trialkyloxy mercapto alkyl silane derivative (II), followed by the addition of the proper silver salt (III). Alternatively, the process of the invention can be carried out by adding to a Ti02 suspension the ligand-silver [L-Ag⁺] adduct, being preformed by reacting the ligand precursor (II) and the silver salt (III) dissolved in a lower alcohol (intended as an alcohol having from 1 to 4 carbon atoms), preferably methanol.
As denoted in the herein below experimental part (see e.g. Examples 1 and 2), both the above indicated synthetic routes allow the preparation of the present derivatives (I) in high yields, and with a quick and cheap methodology, thus enabling the realization of the present process even on industrial scale.

As heretofore reported, the present derivatives (I) can be used as medicament, preferably as antiviral, antimycotic or, even more preferably, as antibacterial agent.
The present derivatives are preferably for the use in the treatment of: cutaneous irritations, inflammations, abrasions, excoriations and/or burns. In a still preferred embodiment, the derivatives of the invention are used in the treatment of diseases selected from: acne, herpes, bed sores, and skin ulcers, preferably decubitus ulcers.
In this respect, in fact, when the derivative are used as medical device, they can be applied to the interested site to form an efficacious film or a barrier, against external microbes, substantially avoiding any infection on the affected skin.
The derivatives are also suitable for the use as a skin cicatrizating agent.
When the derivatives (I) are employed as antibacterial agent, it can be preferably used for the treatment of diseases due to at least one of the microbes selected from: HSV-1 (Herpes Simplex Virus-1), Adenovirus, Poliovirus, Pseudomonas aeruginosa, Enterococcus faecalis, Escherica coli, Salmonella enteridis D1, Listeria monocytogenes, Staphylococcus aureus MR, Staphylococcus aureus MS, Streptococcus pyogenes, Streptococcus salivarius, Streptococcus mitis, Candida albicans, Aspergillus niger.
In the Example 4 of the present experimental part, some preliminary tests have been carried out by the Applicant against a series of microbic species, by using the preferred TiO₂-MPS-Ag⁺ derivative. The results clearly confirm the antibacterial activity of the derivatives of the invention, as also illustrated in Fig. 1a and Fig. 1b. From the considered Figures it is apparent not only the potential antimicrobic activity of the derivatives of the invention, but also the increasing of the activity depending to the amount of silver cations. The grey shadow zone around the deposed material, in fact, is broader in Fig 1b, where derivative (I) with a higher content of Ag+ has been tested. By comparison, Fig. 1C shows that when the TiO₂ powder is used, no activity has been detected.

In another aspect, the present invention also relates to a pharmaceutical composition comprising the above indicated derivatives of formula (I), in admixture with at least one physiologically acceptable excipient or carrier, and optionally, with at least one active ingredient. Preferably said at least one active ingredient is one or more antibacterial agent, even more preferably selected from:

| **Chemical formula** | **Chemical name** |
|---|---|
| 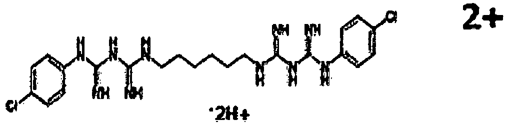 | Chlorhexidine cation |
| 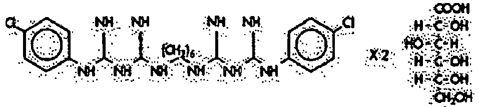 | Chlorhexidine digluconate |
| 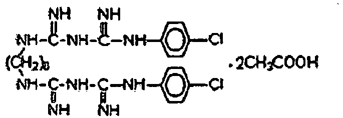 | Chlorhexidine acetate |
| 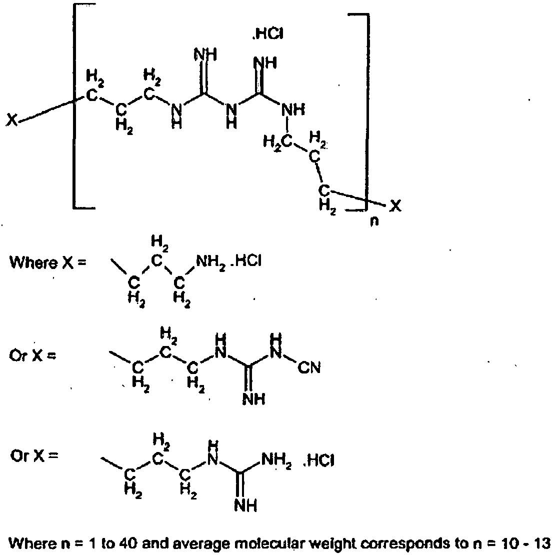 | Polyexamethylene Biguanide hydrochloride Cation (CAS No.32289-58-0) |
| 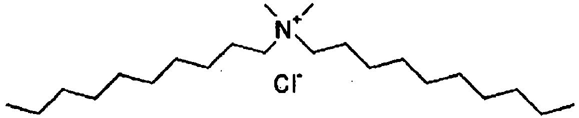 | Didecyl Dimethylammonium Cation |

The physiologically acceptable carriers and/or excipient of the present pharmaceutical compositions, can be selected among those commonly used in the art. As general examples, when the composition is in the form of a cream, said excipients can be suitably selected from: vaseline, liquid paraffin, stearyl alcohol, polyethylene glycol stearate and the like. Similarly, if the composition is in the form of a gel, the derivatives of the invention can be admixed with, e.g., glycerine, amidopropylene glycol, magnesium or aluminium silicate and the like.
It follows that the present derivatives can also be added to known active ingredient, e.g. which have been reported to promote wound healing by increasing collagen content and granulation tissue, such as famotidine or nizadine (for a general reference see e.g. K.S. Rao et al. Indian J. Med. Res. 125, Feb 2007, 149-154).
Owing also to this peculiarity of the present derivatives of formula (I), it is possible to obtain topical-treatment products having antiviral, antibacterial, and antimycotic properties, together with other curative properties, basically depending on the pharmaceutical profile of the drug or composition they are added to.
Therefore, according to a further aspect, the anionic derivatives (I), are also useful as additives for pharmaceutical compositions, thus allowing for instance the preparation of very efficacious topical-treatment products, having multiple or enhanced pharmaceutical properties. Moreover, in the preparation of a pharmaceutical composition of the invention, it is not required any particular precautions when compared to the commonly used processes for preparing topical-treatment products. Hence, by providing the adding phase for adding the present derivatives of formula (I), known processes for producing topical-treatment products may be used to prepare topical-treatment products having antibacterial, antimycotic, virucidal and/or sporicidal properties, comprising the present derivatives (I).
The present compositions for use as medicament are preferably for the use as topical-treatment product, even more preferably in the form of cream, powder, spray gel, gel or foam. Still more preferably, the compositions of the invention can be used for treating: acne, herpes simplex, wounds, decubitus ulcers, cutaneous irritations, inflammations, abrasions, excoriations and/or burns.

Finally, the present invention also refers to a method for treatment of a mammal, including man, comprising the topical administration of an effective amount of a derivative of the above indicated formula (I), or a pharmaceutical composition thereof.

As above described in details, the present invention provides titanium particles where silver ions are covalently bound to the particles surface, via bridging oxygen atoms, through the coupling ligand -[L]- which is also able to coordinate high amount of silver ions Ag⁺. The obtained derivatives can be prepared by means of a simple, quick and cheap synthetic process, also applicable on industrial scale.
Of note, the present derivatives (I) can be used as antimicrobic, antiseptic and/or antiviral agent, in particular in the form of pharmaceutical compositions, such as powders, creams, gels, or foam. Said compositions can also be pharmaceutical formulations where the active specie can act as a barrier being, therefore, very useful for the realization of Medical Devices for topical use, whereby the release of the active silver ions to the tissue should be retained or somehow controlled.

The present invention will now be described by way of examples, which are intended to better illustrate the invention.

### EXPERIMENTAL PART

### Example 1: Preparation of TiO₂-MPS-Ag (Procedure A)

A 100 g amount of TiO₂ (Degussa P25) was suspended under stirring in Acetone (900 ml), followed by addition of 1.06 g of 3-mercaptopropyltrimethoxysilane at room temperature. After 10 minutes AgClO₄ (1.14 g) dissolved in Acetone (100 ml) were added and the stirring continued for about 10 minutes. A precipitate occurs, and the solid product thus obtained was filtered off, and dried at 50 °C.
Elemental analysis, by atomic spectroscopy, confirmed the quantitative formation of the TiO₂-MPS-Ag⁺ derivative, having an Ag⁺ content of about 0.55%.
Higher concentration of Silver ions can be bound to the metal oxide surface following the same procedure, increasing the amount of 3-mercaptopropyltrimethoxysilane and AgClO₄ which were used in stoichiometric amount.

### Example 2: Preparation of TiO₂-MPS-Ag (Procedure B)

AgNO₃ (0.93 g) was dissolved in methanol and 3-mercaptopropyltrimethoxysilane (1.06 g) was added under stirring, to instantaneously form the 3-mercaptopropyltrimethoxysilane-Ag adduct. The solution containing the adduct was added to a suspension of TiO₂ (100 g, Degussa P25) in acetone (900 ml). Stirring was maintained for about 20 minutes, and the solid thus formed was filtered off and dried at 50°C.
Elemental analysis of Silver, by atomic spectroscopy, confirmed the quantitative formation of TiO₂-MPS-Ag+ derivative, with an Ag+ content of about 0.58%.

By varying the amount of silver salt and mercapto trimethoxyalkilsilane derivatives, by both procedures A and B it was possible to obtain TiO₂ powders containing up to a 3 % of Silver ions stabilized on the surface by the MPS ligand.

### Comparative Example 3:

The same procedures of Example 1 and 2 were carried out using the same molar amount of SiO2 in place of TiO2. Under the same experimental conditions (e.g. time, temperature, molar ratio of the reagents) of Example 1 and 2 the procedure with SiO₂ did not lead to the formation of the precipitate comprising the corresponding functionalized derivative.

### Example 4: Antibacterial activity of the TiO₂-MPS-Ag powder.

The antibacterial activity of the powder TiO₂-MPS-Ag powder prepared according to Example 1 or 2, was tested against the following microbic species, purchased from Diagnostic International Distribution SpA:

| | |
|---|---|
| Pseudomonas aeruginosa | ATCC 15442 |
| Staphylococcus aureus | ATCC 6538 |
| Escherichia coli | ATCC 10536 |
| Enterococcus hirae | ATCC 10541 |
| Candida albicans | ATCC 10231 |

A mixture of the different microorganism with concentrations in the range 1.5x107 - 5.5x107 for each species was prepared.
100 µl of the mixture was seeded in Petri capsule containing Tryptone Soya Agar.
Samples of TiO₂-MPS-Ag powders having respectively 0.58% and 2.30% Ag⁺ content prepared according to example 1 and 2, and control powders without silver cations, were put inside the Petri capsule which were then incubated at 37° C for 24 h.
After this time the capsule were examined evaluating the colonies proliferation and the inhibition ring surrounding the antibacterial powder.
As shown in Figure 1, the thickness of the inhibition ring surrounding the TiO₂-MPS-Ag powders was observed to be larger the higher was the Ag⁺ content (see Fig. 1A where 0.58% Ag+ composition were used, Fig. 1B where 2.30% Ag+ composition were used), while no inhibition ring was observed on the control sample of TiO₂ powder (see Fig. 1C).

## Claims

1. A derivative of formula (I):
**TiO₂-[L]-Ag⁺** **(I)**
wherein:
-[L]- is a bivalent mercapto alkyl silane ligand of general formula: -[Si-R-S]-, wherein R is a bivalent C₁-C₄ linear or branched alkyl group, optionally substituted;
TiO₂ represent a titanium dioxide particle linked to the silica atom of the ligand -[L]-;
Ag⁺ represents silver ions coordinated to the sulphur atom of the bivalent ligand -[L]-.

2. The derivative of formula (I) according to claim 1, **characterized by** the fact that the titanium dioxide particle has an average diameter comprised from 100 to 600 nm, preferably comprised from 300 to 500nm.

3. The derivative of formula (I) according to any one of claims 1-2, wherein R is -CH₂- or -CH₂CH₂CH₂-.

4. The derivative of formula (I) according to claim 1, having an amount of silver ions comprised from 0.5 to 3% w/w.

5. The derivative of formula (I) according to any one of the preceding claims, for use as a medicament.

6. The derivative of formula (I) for use according to claim 5, as antibacterial, antimycotic, antiviral or sporicidal agent.

7. The derivative of formula (I) for use according to claim 5 or 6, in the treatment of cutaneous irritations, inflammations, abrasions, excoriations and/or burns.

8. The derivative of formula (I) for use according to any one of claim 5 to 7, in the treatment of: acne, herpes, bed sores, and skin ulcers, preferably decubitus ulcers.

9. The derivative of formula (I) for use according to any one of claim 5 to 8, as a skin cicatrizating agent.

10. The derivative of formula (I), for use according to any one of claim 5 to 9, as antibacterial agent for treatment of diseases due to at least one of the microbes selected from: HSV-1 (Herpes Simplex Virus-1), Adenovirus, Poliovirus, Pseudomonas aeruginosa, Enterococcus faecalis, Escherichia coli, Salmonella enteritidis D1, Listeria monocytogenes, Staphylococcus aureus MR, Staphylococcus aureus MS, Streptococcus pyogenes, Streptococcus salivarius, Streptococcus mitis, Candida albicans, Aspergillus niger.

11. A pharmaceutical composition comprising the derivative of formula (I) as described in any one of claim 1 to 4, in admixture with at least one physiologically acceptable excipient and/or carrier, and, optionally, with at least another pharmaceutical active ingredient.

12. The pharmaceutical composition according to claim 11, wherein said at least another pharmaceutical active ingredient is selected from: Chlorhexidine cation, Chlorhexidine digluconate, Chlorhexidine acetate, Polyexamethylene Biguanide hydrochloride Cation, Didecyl Dimethylammonium Cation.

13. The pharmaceutical composition according to claims 11 or 12, in the form of a cream, a gel, a foam or a powder.

14. The pharmaceutical composition according to claims 11 to 13, for topic administration.

15. A process for the preparation of the present derivatives of formula (I) as defined in any one the claim 1-4, said process comprising the reaction of the titanium dioxide with a trialkyloxy mercaptosilane derivative of formula (II), in the presence of a silver salt (III): 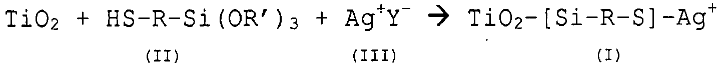 wherein:
R is as defined in claims 1, 3-4;
R' is independently selected from C₁-C₄ monovalent linear or branched alkyl group, optionally substituted, preferably methyl;
Y⁻ is an anionic counterion, preferably selected from: perchlorate, acetate and nitrate.

## Patentansprüche

1. Derivat der Formel (I):
TiO₂-[L]-Ag⁺ (I)
wobei:
-[L]- ein bivalenter Mercapto-Alkyl-Silan-Ligand der allgemeinen Formel ist: -[Si-R-S]-, wobei R eine bivalente C₁-C₄ lineare oder verzweigte Alkylgruppe ist, optional ersetzt;
TiO₂ ein Titaniumdioxidpartikel verbunden mit dem Siliziumatom des Ligands -[L]- darstellt;
Ag⁺ Silberionen koordiniert mit dem Schwefelatom des bivalenten Ligands -[L]- darstellt.

2. Derivat der Formel (I) nach Anspruch 1, **dadurch gekennzeichnet, dass** das Titaniumdioxidpartikel einen durchschnittlichen Durchmesser von 100 bis 600 nm hat, vorzugsweise zwischen 300 und 500 nm.

3. Derivat der Formel (I) nach einem der Ansprüche 1-2, wobei R -CH₂- oder -CH₂CH₂CH₂- ist.

4. Derivat der Formel (I) nach Anspruch 1, mit einem Anteil an Silberionen zwischen 0,5 und 3 Gew.%.

5. Derivat der Formel (I) nach einem der vorhergehenden Ansprüche, zur Verwendung als ein Medikament.

6. Derivat der Formel (I) zur Verwendung nach Anspruch 5, als antibakterieller, antimykotischer, antiviraler oder sporentötender Wirkstoff.

7. Derivat der Formel (I) zur Verwendung nach Anspruch 5 oder 6, bei der Behandlung von Hautreizungen, Entzündungen, Abschürfungen, Exkoriationen bzw. Verbrennungen.

8. Derivat der Formel (I) zur Verwendung nach einem der Ansprüche 5 bis 7, bei der Behandlung von: Akne, Herpes, Liegengeschwüren, und Hautgeschwüren, vorzugsweise Dekubitaulzera.

9. Derivat der Formel (I) zur Verwendung nach einem der Ansprüche 5 bis 8, als hautvernarbender Wirkstoff.

10. Derivat der Formel (I), zur Verwendung nach einem der Ansprüche 5 bis 9, als antibakterieller Wirkstoff bei der Behandlung von Krankheiten aufgrund von mindestens einer Mikrobe ausgewählt aus: HSV-1 (Herpes Simplex Virus-1), Adenovirus, Poliovirus, Pseudomonas aeruginosa, Enterococcus faecalis, Escherichia coli, Salmonella enteritidis D1, Listeria monocytogenes, Staphylococcus aureus MR, Staphylococcus aureus MS, Streptococcus pyogenes, Streptococcus salivarius, Streptococcus mitis, Candida albicans, Aspergillus niger.

11. Pharmazeutische Rezeptur umfassend das Derivat der Formel (I), wie beschrieben in einem der Ansprüche 1 bis 4, in Beimischung mit mindestens einem physiologisch zulässigen Trägerstoff bzw. Überträger, und optional, mit mindestens einem anderen pharmazeutisch aktivem Inhaltsstoff.

12. Pharmazeutische Rezeptur nach Anspruch 11, wobei der mindestens eine andere pharmazeutisch aktive Inhaltsstoff ausgewählt wird aus: Chlorhexidin Kation, Chlorhexidindiglukonat, Chlorhexidinazetat, Polyhexamethylenbiguanidhydrochlorid Kation, Didecyldimethylammonium Kation.

13. Pharmazeutische Rezeptur nach Anspruch 11 oder 12, in der Form einer Creme, eines Gels, eines Schaums oder eines Puders.

14. Pharmazeutische Rezeptur nach Anspruch 11 bis 13, für topische Verabreichung.

15. Herstellungsverfahren der vorliegenden Derivate der Formel (I) wie definiert in einem der Ansprüche 1-4, wobei genanntes Verfahren die Reaktion des Titaniumdioxids mit einem Trialkyloxy-Mercaptosilan-Derivat der Formel(II) umfasst, in Gegenwart eines Silbersalzes (III) : 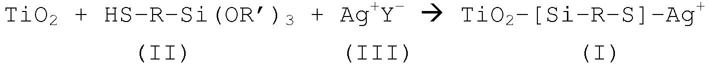 wobei:
R wie definiert in Ansprüchen 1, 3-4 ist;
R' unabhängig ausgewählt aus C₁-C₄ monovalenter linearer oder verzweigter Alkylgruppe ist, optional ersetzt, vorzugsweise Methyl;
Y⁻ ein anionisches Gegenion, vorzugsweise ausgewählt ist aus: Perchlorat, Azetat und Nitrat.

## Revendications

1. Dérivé de formule (I) :
TiO₂-[L]-Ag⁺ (I)
dans lequel :
-[L]- est un ligand silane alkyle mercapto bivalent de formule générale : -[Si-R-S]-, dans lequel R est un groupe alkyle linéaire ou ramifié bivalent en C₁-C₄, éventuellement substitué ;
TiO₂ représente une particule de dioxyde de titane liée à l'atome de silice du ligand -[L]- ;
Ag⁺ représente les ions argent coordonnés à l'atome de soufre du ligand bivalent -[L]-.

2. Dérivé de formule (I) selon la revendication 1, **caractérisé par le fait que** la particule de dioxyde de titane possède un diamètre moyen compris entre 100 et 600 nm, de préférence compris entre 300 et 500 nm.

3. Dérivé de formule (I) selon l'une quelconque des revendications de 1 à 2, dans lequel R est -CH₂- ou - CH₂CH₂CH₂-.

4. Dérivé de formule (I) selon la revendication 1, ayant une quantité d'ions argent compris entre 0,5 et 3% M/M.

5. Dérivé de formule (I) selon l'une quelconque des revendications précédentes, à utiliser comme médicament.

6. Dérivé de formule (I) à utiliser selon la revendication 5, comme antibactérien, antimycotique, antiviral ou agent sporicide.

7. Dérivé de formule (I) à utiliser selon les revendications 5 ou 6, dans le traitement des irritations cutanées, des inflammations, des éraflures, des excoriations et/ou des brûlures.

8. Dérivé de formule (I) à utiliser selon l'une quelconque des revendications de 5 à 7, dans le traitement de : l'acné, de l'herpès, des escarres de décubitus et des ulcères de la peau, de préférence des plaies de lit.

9. Dérivé de formule (I) à utiliser, selon l'une quelconque des revendications de 5 à 8, comme agent cicatrisant de la peau.

10. Dérivé de formule (I) à utiliser, selon l'une quelconque des revendications de 5 à 9, comme agent antibactérien pour le traitement de maladies provoquées par au moins l'une des bactéries sélectionnées parmi celles qui suivent : HSV-1 (virus herpès simplex de type 1), Adénovirus, Poliovirus, Pseudomonas aeruginosa, Enterococcus faecalis, Escherichia coli, Salmonella enteritidis D1, Listeria monocytogenes, Staphylocoque doré MR, Staphylocoque doré MS, Streptocoque pyogène, Streptococcus salivarius, Streptococcus mitis, Candida albicans, aspergillus niger.

11. Composition pharmaceutique, comprenant le dérivé de formule (I) tel que décrit dans l'une quelconque des revendications de 1 à 4, mélangée avec au moins un excipient et/ou un vecteur de médicament physiologiquement acceptable, et, éventuellement avec au moins un ingrédient pharmaceutique actif supplémentaire.

12. Composition pharmaceutique selon la revendication 11, dans laquelle ledit au moins un autre ingrédient pharmaceutique actif supplémentaire est sélectionné à partir de : cation de chlorhexidine, digluconate de chlorhexidine, acétate de chlorhexidine, cation de chlorhydrate de poly-hexaméthylène Biguanide, cation de didécyldiméthylammonium.

13. Composition pharmaceutique selon les revendications 11 ou 12, sous forme de crème, de gel, de mousse ou de poudre.

14. Composition pharmaceutique selon les revendications 11 à 13, destinée à une administration topique.

15. Procédé de préparation du présent dérivés de formule (I) tel que définis dans l'une quelconque des revendications de 1 à 4, ledit procédé comprenant la réaction du dioxyde de titane avec un dérivé de formule (II) de mercaptosilane de trialkyloxy, en présence d'un sel d'argent (III) : 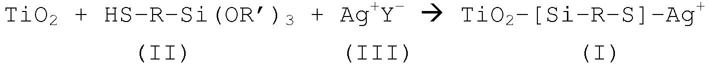 dans lequel :
R est tel que défini dans les revendications 1, 3 et 4 ;
R' est indépendamment sélectionné à partir d'un groupe alkyl linéaire ou ramifié monovalent en C₁-C₄, éventuellement substitué, de préférence par du méthyle ;
Y⁻ est un contre-ion anionique, de préférence sélectionné à partir de : perchlorate, d'acétate et de nitrate.
